# FASCICULE DE BREVET EUROPEEN

(11) **EP 3 528 643 B1**
(45) Date de publication et mention de la délivrance du brevet: **04.11.2020**
(21) Numéro de dépôt: 17783931.3
(22) Date de dépôt: 21.09.2017
(51) Int. Cl.: A23L 3/04, A23L 5/10

(54) **DISPOSITIF DE TRAITEMENT PAR CUISSON DE PRODUITS**
ANLAGE ZUR VERARBEITUNG DURCH KOCHEN VON PRODUKTEN
APPARATUS FOR THE TREATMENT BY COOKING OF PRODUCTS

(30) Priorité: 21.10.2016 FR 1660248
(43) Date de publication de la demande: 28.08.2019
(73) Titulaire: CPA Holding, 67700 Saverne (FR)
(72) Inventeur: CAMU, Patrice, 67700 Saverne (FR)
(74) Mandataire: Cabinet Bleger-Rhein-Poupon
(86) Numéro de dépôt international: PCT/FR2017/052532
(87) Numéro de publication internationale: WO 2018/073506

(56) Documents cités:
- WO-A1-90/04928
- FR-A1- 3 023 169
- US-A1- 2005 074 532
- US-A1- 2011 250 335
- US-A1- 2014 125 482
- US-A1- 2015 106 679

## Description

La présente invention entre dans le domaine du traitement par cuisson, stérilisation et/ou pasteurisation de produits alimentaires.

On notera que de tels produits peuvent se présenter nus, sans emballage, ou bien emballés au sein d'un contenant ouvert ou clos hermétiquement, ledit contenant pouvant être rigide, semi-rigide ou souple.

L'invention vise tout particulièrement le suivi du traitement subi par chaque produit ou groupe de produits, de manière à déterminer de façon exacte les conditions de cuisson, stérilisation et/ou pasteurisation subies par chaque produit ou groupe de produits, ainsi qu'améliorer leur traçabilité.

Au sens de la présente invention, dans un souci de clarté, on entend génériquement par le terme « cuisson », sauf spécifié autrement, la cuisson, la stérilisation et/ou la pasteurisation d'au moins un produit alimentaire. On entend génériquement par le terme « produits », sauf spécifié plus précisément, un produit ou un groupe de plusieurs produits, tandis que la totalité d'un type de produits subissant la même cuisson consiste en un « lot de produits ».

De manière connue, industriellement, la cuisson de produits alimentaires s'effectue selon un cycle comprenant au moins une étape de chauffage et au moins une étape de refroidissement. Ces étapes se déroulent au sein de dispositifs adaptés, comprenant réciproquement au moins un module de chauffage et au moins un module de refroidissement. Des dispositifs automatisés de cuisson sont connus des documents US 2005/0074532 et US 2015/ 0074532, par exemple.

On notera que, dans le cadre de la présente invention, le cycle de cuisson s'effectue de façon continue, à savoir que des produits sont acheminés, au travers de moyens de convoyage adaptés, en un flux continu ou pas à pas, depuis une entrée située en amont du premier module de chauffage vers une sortie située en aval du dernier module de refroidissement, lesdits moyens de convoyage traversant successivement lesdits modules de chauffage et de refroidissement depuis l'entrée jusqu'à la sortie. Les produits peuvent être disposés directement sur les moyens de convoyage, comme dans le cas d'un convoyeur de type tapis roulant, accrochés ou suspendus dans le cas d'une crémaillère, ou encore posés sur un support comme une plaque ou entreposés au sein d'un panier support, lui-même reposant ou étant accroché auxdits moyens de convoyage.

De surcroît, les atmosphères internes des modules de chauffage et de refroidissement peuvent être soumises à une pression destinée à contrer l'augmentation de volume du produit générée par l'élévation de température, plus particulièrement dans le cas de produits sous emballage souple fermé hermétiquement. La dilatation du produit sous l'effet de la chaleur provoque l'expansion et un risque de rupture dudit emballage : il est donc nécessaire d'appliquer une pression pour limiter l'expansion de l'emballage et empêcher sa détérioration. Dès lors, les modules de chauffage et de refroidissement peuvent être équipés de sas de mise sous pression au niveau des entrées et sorties. Ainsi, même lors d'un cycle continu, l'introduction et l'extraction des produits à travers les sas d'entrée et de sortie nécessitent l'avancement pas à pas des produits.

Plus précisément, de manière connue, il existe des dispositifs dont les modules s'étendent horizontalement et d'autres dont les modules s'étendent verticalement, sous forme de colonnes. Au sens de la présente invention, chaque module de chauffage ou de refroidissement se présente sous la forme d'au moins une colonne s'étendant verticalement.

Chaque colonne comprend des moyens de chauffage ou de refroidissement répartis sur sa hauteur, définissant des sections correspondant à des étapes distinctes du cycle de cuisson. Notamment, des moyens de chauffage peuvent se présenter sous la forme de buses d'injection, vers l'intérieur de la colonne de chauffage, d'eau chaude à des températures différentes en fonction de la section où lesdites buses se situent et la température à appliquer aux produits en fonction de la cuisson voulue. A titre d'exemple, les buses inférieures peuvent injecter une eau à une température moindre que les buses supérieures, assurant ainsi graduellement une montée en température des produits circulant du bas vers le haut de la colonne de chauffage. Chaque module de refroidissement peut fonctionner d'une façon similaire, avec de l'eau froide.

En outre, chaque module comprend des moyens de gestion permettant de contrôler de façon précise les conditions de cuisson à chaque étape du traitement, à savoir de connaître les conditions appliquées au niveau de chacune desdites sections, comme par exemple le débit et la température de l'eau injectée. Ces moyens de gestion permettent aussi de contrôler la vitesse d'avancement des moyens de convoyage.

Un inconvénient majeur réside dans le fait qu'il est difficilement possible d'identifier de façon certaine le traitement que subissent les produits, notamment en raison de la difficulté d'identifier un produit ou un groupe de produits parmi la totalité des produits qui subissent le traitement de cuisson depuis le démarrage jusqu'à l'arrêt du dispositif (à savoir le lot de produits qui, lui, peut être identifié en apposant notamment un numéro unique identique sur tous les emballages). En effet, pour des questions économiques et pratiques, si la totalité des produits d'un lot peut être identifiée, il est difficilement envisageable d'apposer sur chaque produit ou groupe de produits, ou bien sur leur emballage, au moment de leur séparation un par un ou bien en groupe, un numéro unique d'identification supplémentaire.

De façon connexe, un autre inconvénient réside dans l'écoulement de l'eau utilisée pour chauffer ou refroidir les produits lors de leur passage au niveau des différentes sections. En effet, l'eau d'une section supérieure, à une température donnée, se refroidit ou se réchauffe au contact des produits réciproquement à chauffer ou à refroidir, puis s'écoule naturellement vers le bas vers une section inférieure, modifiant les conditions réelles de cuisson des produits traversant une section située inférieurement. Même en contrôlant et connaissant précisément les conditions appliquées à chaque section, il n'est alors pas possible de savoir avec exactitude quel traitement ont reçu les produits. En effet, en dehors des premiers à subir le cycle, les produits suivants peuvent subir un traitement altéré, susceptible de ne pas aboutir à une cuisson voulue, voire à obtenir une stérilisation et/ou pasteurisation incomplète.

L'invention a pour but de pallier les inconvénients de l'état de la technique en proposant une traçabilité par produit ou groupe de produits d'un lot complet lors d'un traitement de cuisson en continu, notamment pas à pas, au sein d'un dispositif de cuisson, sans nécessité d'apposer d'éléments supplémentaires sur lesdits produits. L'invention vise ainsi à assurer un suivi amélioré de la cuisson de chaque produit ou groupe de produits.

Pour ce faire, la présente invention envisage d'effectuer le suivi des produits d'un lot, un par un ou bien par groupe, en identifiant, non pas lesdits produits, mais le support servant à leur transport via les moyens de convoyage. Dès lors, en connaissant les conditions au niveau de chaque section des modules de chauffage et de refroidissement, ainsi que la vitesse d'avancement des moyens de convoyage, il est possible de déterminer avec exactitude la position à un instant donné de chaque support et, de ce fait, le traitement subi à chaque étape pour les produits positionnés au niveau de chaque support.

La traçabilité des produits au sein de l'installation est alors grandement améliorée, permettant de détecter tout changement réellement survenu lors de chaque étape du traitement par cuisson des produits. En cas d'anomalie constatée, il est alors possible d'extraire directement les produits concernés en sortie du dispositif.

De façon complémentaire et subsidiaire, chaque étape du traitement est améliorée au travers de supports sous forme de plaques pleines, sur lesquelles sont disposés les produits et qui sont identifiées comme susmentionné. Ces plaques pleines sont pourvues de moyens assurant un écoulement latéralement, en particulier de moyens de canalisation latérale. Dès lors, au sein des colonnes d'un dispositif de traitement par cuisson selon l'invention, des moyens de canalisation latérale présents sur une plaque située au niveau d'une section aspergée par de l'eau à une température donnée, guident latéralement l'eau vers les côtés de ladite plaque, après son contact avec les produits y positionnés. Ainsi, lesdits moyens de canalisation latérale de la plaque dérivent l'écoulement de l'eau de sorte qu'elle ne rencontre pas les plaques situées inférieurement, au niveau d'une même section ou d'une section située plus bas dans la colonne.

A cet effet, tout d'abord, l'invention concerne un procédé de traitement par cuisson de produits, dans lequel des produits subissent un cycle de cuisson sous forme d'au moins une étape de chauffage au sein d'au moins un module de chauffage, puis d'au moins une étape de refroidissement au sein d'au moins un module de refroidissement, lesdits produits étant disposés sur des supports convoyés successivement au travers desdits modules de chauffage et de refroidissement, ledit procédé consistant à gérer les conditions de cuisson de chaque étape de chauffage et de refroidissement en contrôlant au moins la température au niveau d'au moins une section de chaque module et la vitesse de convoyage desdits supports, caractérisé en ce que :
- on identifie de façon unique chaque support ;
- on enregistre les conditions de cuisson subies par chaque support identifié à chaque étape dudit cycle, en fonction de ladite vitesse de convoyage ;
- on détermine les conditions de cuisson subies par les produits positionnés sur chaque support identifié.

Ainsi, en positionnant les produits d'un lot, un par un ou bien par groupe, sur un support identifié, il est possible d'extrapoler à chaque instant du cycle de cuisson, en fonction de la vitesse de défilement du convoyeur, où se situent chaque support et les produits qu'il transporte. Dès lors, en connaissant les conditions liées aux étapes de cuisson, au niveau de chaque section de l'installation, il est possible de déterminer en temps réel, avec exactitude, le traitement subi par lesdits produits.

L'invention concerne aussi un dispositif pour la mise en œ uvre d'un tel procédé, comprenant au moins un module de chauffage et au moins un module de refroidissement, ainsi que des moyens de convoyage desdits produits au travers desdits modules, lesdits moyens de convoyage comprenant des supports sur lesquels sont positionnés lesdits produits, lesdits modules se présentant sous la forme d'au moins une colonne et comprenant réciproquement des moyens de chauffage et de refroidissement situés au niveau des sections de chaque colonne, ledit dispositif comprenant encore des moyens de gestion des conditions de cuisson de chacun desdits moyens de chauffage et de refroidissement, ainsi que de gestion de la vitesse d'avancement desdits moyens de convoyage, caractérisé par le fait que
- chaque support comprend un identifiant unique ;
- lesdits moyens de gestion comprennent des moyens d'enregistrement des conditions de cuissons subies par chaque support au niveau de chaque section, en fonction de ladite vitesse d'avancement ;
- lesdits moyens de gestion comprenant des moyens de détermination des conditions de cuisson subies par lesdits produits positionnés sur chaque support.

Selon une autre caractéristique du dispositif de l'invention, lesdits moyens de chauffage et de refroidissement consistent en au moins des buses d'injection d'eau.

De façon connexe, l'invention améliore la cuisson des produits à chaque étape, en s'assurant que les conditions restent optimales.

En particulier, dans le cas de projection d'eau à une température précise au niveau de chaque section, correspondant à chaque étape, l'invention prévoit de contrôler l'écoulement de l'eau de sorte qu'elle ne vienne pas au contact des produits situés inférieurement, au niveau d'au moins une section plus basse, en train de subir une autre étape dudit traitement.

De façon subsidiaire, l'invention vise l'utilisation de supports assurant l'écoulement susmentionné au sein d'un dispositif selon l'invention, de manière à empêcher l'eau d'une section à venir perturber les conditions de cuisson d'une section inférieure.

Plus spécifiquement, dans l'invention, l'écoulement d'eau est assuré par des moyens de canalisation latérale présents sur lesdits supports des produits à traiter par cuisson.

De façon encore subsidiaire, l'invention vise l'utilisation de supports numérotés de façon unique pour obtenir la traçabilité assurée lors de la mise en œuvre du procédé de traitement par cuisson selon l'invention.

D'autres caractéristiques et avantages de l'invention ressortiront de la description détaillée qui va suivre des modes de réalisation non limitatifs de l'invention, en référence aux figures annexées dans lesquelles :
- la figure 1 représente schématiquement, en perspective, un mode non limitatif de réalisation d'un dispositif de traitement par cuisson selon l'invention, pourvu d'un seul module de chauffage suivi des trois modules de refroidissement, avec un circuit de retour des supports ; et
- la figure 2 représente une vue schématisée selon une coupe verticale d'un détail de la figure 1, montrant l'entrée dudit module de chauffage et le cheminement des supports identifiés.

La présente invention concerne le traitement par cuisson de produits, à savoir un par un ou par groupe parmi un lot complet de produits.

Un tel traitement consiste en un cycle de cuisson sous forme d'au moins une étape de chauffage suivie d'au moins une étape de refroidissement. Plusieurs étapes de chauffage et/ou de refroidissement peuvent être envisagées en fonction de la cuisson souhaitée et du type de produits à traiter.

Un tel cycle intervient au sein d'un dispositif 1 adapté, comprenant au moins un module de chauffage 2 et au moins un module de refroidissement 3. Plusieurs modules de chauffage 2 et/ou de refroidissement 3 peuvent se succéder. Les modules de chauffage 2 et de refroidissement 3 peuvent être reliés, à savoir que les produits peuvent passer de l'un à l'autre, en particulier depuis le dernier module de chauffage 2 vers le premier module de refroidissement 3.

Selon un mode de réalisation particulier, non limitatif, au moins un module intermédiaire 4 peut se situer entre le dernier module de chauffage 2 et le premier module de refroidissement 3, permettant d'apporter un traitement complémentaire auxdits produits, comme par exemple un pré-refroidissement ou bien une modification de pression entre les modules 2, 3.

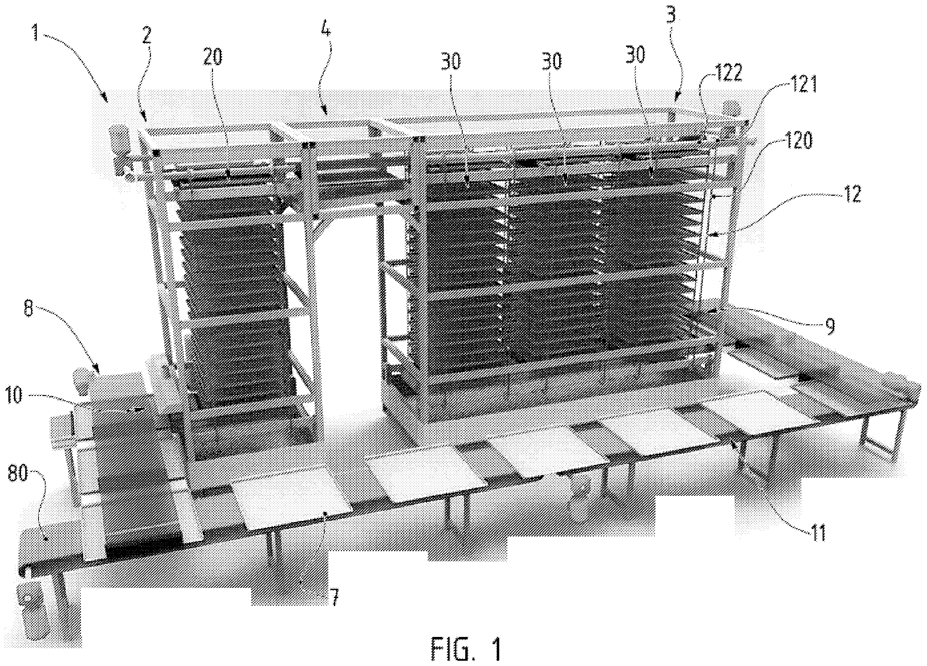

Selon l'exemple de réalisation visible sur la figure 1, un unique module de chauffage 2 est relié par un seul module intermédiaire 4 à un module de refroidissement 3.

De façon non limitative, le traitement peut prévoir d'appliquer au sein d'un module 2, 3 une pression destinée à compenser l'élévation de température des produits lors de leur traitement, évitant notamment l'expansion et la détérioration de leur emballage éventuel ou tout simplement du produit lui-même. En outre, le dispositif peut alors comprendre, en entrée au niveau du premier module de chauffage 2, ainsi qu'en sortie au niveau du dernier module de refroidissement 3, des sas de mise sous pression.

Par ailleurs, une ou plusieurs étape(s) de chauffage ou de refroidissement peut/peuvent être réalisée(s) au sein de plusieurs ou d'un même module 2, 3 correspondant. Plus précisément, plusieurs étapes de chauffage ou de refroidissement successives peuvent être réalisées le long d'un même module 2, 3. Les produits peuvent alors subir, dans un même module de chauffage 2 ou de refroidissement 3, réciproquement une montée ou une descente de température, voire un maintien à une température définie.

Pour ce faire, le dispositif 1 selon l'invention comprend des moyens 5 de chauffage et de refroidissement répartis le long de chaque module réciproquement de chauffage 2 et de refroidissement 3. Ces moyens 5 permettent de chauffer et refroidir les produits à des températures définies, identiques ou différentes, en des sections 6 distinctes dudit dispositif 1. Dès lors, chaque section 6 peut correspondre à une étape du cycle. Plusieurs sections 6 peuvent aussi correspondre à une même étape.

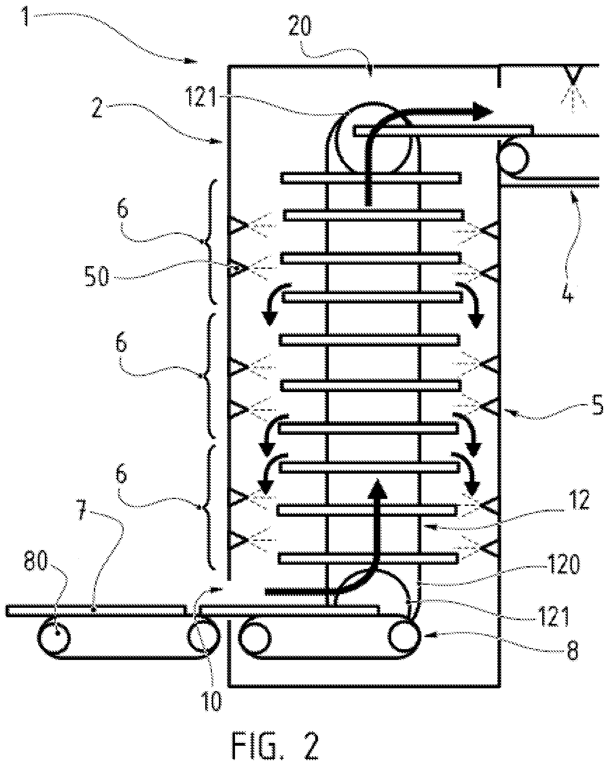

De telles sections 6 ont été mises en évidences sur l'exemple visible sur la figure 2.

Afin d'acheminer les produits d'un module 2, 3 à un autre, l'invention prévoit de les disposer sur des supports 7 et de convoyer ces supports 7 successivement au travers desdits modules de chauffage 2 et de refroidissement 3.

Pour ce faire, ledit dispositif 1 comprend des moyens de convoyage 8 adaptés auxdits produits. Comme évoqué précédemment, les moyens de convoyage 8 comprennent des supports 7, amovibles, recevant en face supérieure lesdits produits. Ces derniers sont positionnés et reposent sur lesdits supports 7. Les moyens de convoyage 8 permettent alors de transporter les supports 7 et les produits qui y sont placés depuis l'extérieur vers l'intérieur, et inversement. Les moyens de convoyages 8 permettent de transporter les supports 7 supportant les produits au sein de tout le dispositif 1, afin que les supports 7 et leurs produits traversent lesdits modules de chauffage 2 et de refroidissement 3.

De façon subsidiaire, lesdits moyens de convoyage 8 permettent aussi d'assurer, en un circuit continu, le retour des supports 7 vidés, une fois les produits retirés après traitement, entre la sortie 9 et l'entrée 10 du dispositif 1, le long d'un circuit retour 11. Un tel circuit 11 est visible sur la figure 1.

Selon l'invention, les modules de chauffage 2 et de refroidissement 3 s'étendent verticalement. Ils se présentent alors sous la forme d'au moins une colonne réciproquement de chauffage 20 et de refroidissement 30.

On notera qu'un même module 2, 3 peut être divisé en une ou plusieurs colonnes 20, 30. Selon l'exemple représenté sur la figure 1, le module de chauffage 2 comprend une unique colonne 20 tandis que le module de refroidissement 3 comprend trois colonnes de refroidissement 30. L'utilisation de colonnes s'étendant verticalement présente comme avantage que l'espace dédié à la cuisson et au traitement encombre uniquement la hauteur du local industriel dans lequel le dispositif se trouve. En conséquence, il existe un gain de place horizontal non négligeable dans ledit local de traitement et de cuisson, ce gain de place constitue à échelle industrielle un gain économique.

Dans ce cadre, lesdits moyens de chauffage et de refroidissement 5 sont répartis à différentes hauteurs au sein de chaque colonne 20, 30, définissant lesdites sections 6 correspondant à une ou plusieurs étapes du cycle du traitement par cuisson.

Selon le mode préférentiel, non limitatif, lesdits moyens de chauffage et de refroidissement 5 peuvent consister au moins en des buses 50 d'injection d'eau. Chaque section 6 peut comprendre une ou plusieurs buses 50. Ces dernières sont positionnées et orientées de manière à projeter de l'eau à une température définie, en fonction de l'étape correspondant à la cuisson souhaitée des produits, vers les supports 7 et les produits qu'ils transportent. De cette manière, chacune des buses 50 appartenant à une même section 6 va injecter de l'eau à une température correspondant à une étape de cuisson souhaitée.

L'exemple représenté sur la figure 2 met en évidence l'eau projetée par plusieurs buses 50.

Dès lors, lesdits moyens de convoyage 8 peuvent être subdivisés en plusieurs portions assurant, d'une part, le transport des supports 7 verticalement, de façon ascendante et/ou descendante au sein de chaque colonne 20, 30, et d'autre part, le transport des supports 7 horizontalement, avant l'entrée 10 et après la sortie 9, ainsi que lors de l'éventuel circuit retour 11.

Plusieurs types de convoyeurs peuvent être envisagés pour le transport vertical des supports 7, notamment sous forme de plusieurs vis sans fin, tournant et assurant l'élévation et la descente à plat de supports 7 plans, notamment sous forme de plaque ou plateau. Une solution alternative préférée consiste en au moins une paire de crémaillères 12 s'étendant verticalement et parallèlement, de part et d'autre de chaque colonne 20, 30. Chaque crémaillère se présente sous forme de chaînes 120 enroulées autour de roues de renvoi 121 en partie haute et basse de chaque colonne 20, 30. Au moins une desdites roues 121 assure l'entraînement de chaque chaîne 120. En particulier, plusieurs roues 120 de plusieurs colonnes 20, 30 peuvent être entraînées par un même arbre d'entraînement 122 autour duquel elles sont positionnées, comme visibles sur la figure 1.

De plus, à intervalles réguliers, les maillons constituant chaque chaîne 120 peuvent comprendre des moyens de réception et de maintien horizontal des supports 7, par exemple sous la forme de taquets ou bras s'étendant orthogonalement par rapport à chaque maillon.

Par ailleurs, pour le transport éventuel horizontalement, les moyens de convoyage 8 peuvent être constitués sur cette portion d'au moins un tapis roulant à bande 80.

En outre, dans le cas de sas aux entrée 10 et sortie 9, des convoyeurs horizontaux à bande ou similaires peuvent être envisagés, notamment sous forme de tapis roulant 80.

Préférentiellement, les supports 7 se présentent alors sous forme de plaques, conformées de manière à coopérer avec lesdits moyens de convoyage 8, en particulier au niveau des sections 6 de transport vertical, à savoir des crémaillères 12.

On notera que la vitesse de progression des supports 7 et donc des produits peut être réglée en fonction de la vitesse d'avancement desdits moyens de convoyage 8.

Ainsi, les produits peuvent être acheminés et subir leur cycle de traitement par cuisson.

A ce titre, l'invention consiste aussi à gérer les conditions de cuisson de chaque étape de chauffage et de refroidissement en contrôlant au moins la température au niveau d'au moins une section 6 de chaque module 2, 3.

Dans le cas de buses 50, l'invention permet au moins de gérer de façon indépendante la température de l'eau en sortie de chaque buse ou groupe de buses 50 d'une même section 6. Les buses 50 d'une même section 6 constituent donc un moyen de contrôle de la température au niveau de chaque section 6.

En conséquence, au niveau de chaque section 6, la température des moyens de chauffage et de refroidissement 5 est totalement contrôlée et définie par la température de l'eau en sortie des buses 50.

De plus, d'autres paramètres, que la température de l'eau en sortie des buses 50, peuvent être régulés pour contrôler les conditions de cuisson au travers des moyens de chauffage et de refroidissement 5. Par exemple, la fréquence et le débit de projection d'eau par les buses 50 peuvent également consister en des moyens de contrôle des conditions de cuisson au niveau de chacune des sections 6. Il est donc possible de configurer et superviser avec exactitude chaque étape du cycle.

Cette gestion peut s'effectuer par des moyens de gestion adaptés, non représentés, de préférence informatique, qu'il est possible de programmer. Cette programmation des moyens de gestion se fera en fonction du cycle et du traitement de cuisson souhaités pour chaque type de produits, mais également de l'installation, à savoir du nombre de modules 2, 3 et leurs colonnes 20, 30 dont est pourvu le dispositif 1. Ces moyens de gestion, avantageusement programmables informatiquement, assurent donc la gestion indépendante des conditions de cuisson de chacun desdits moyens de chauffage et de refroidissement 5.

Avantageusement, afin d'améliorer et d'obtenir une meilleure traçabilité des produits traités, l'invention prévoit d'identifier de façon unique chaque support 7. Ainsi, au lieu de marquer chaque produit ou groupe de produits, on les dispose sur un support 7 déterminé et connu de façon certaine, permettant de façon virtuelle et pour le temps du cycle, d'affecter une identification unique aux produits disposés sur chaque support 7 répertorié.

On notera que cette identification de chaque support 7 peut s'effectuer de façon physique, en apposant ou inscrivant des données d'identification sur chaque support 7. L'identification peut aussi être virtuelle, à savoir que les supports 7 ne présentent pas d'inscription, mais l'identification consiste en un ordonnancement, à savoir leur ordre sur le circuit de l'installation, à savoir le long des moyens de convoyage 8. Cette dernière solution est particulièrement adaptée lorsque le convoyage des supports 7 s'effectue de façon fermée, avec un circuit retour 11, comme visible sur la figure 1.

En outre, l'invention permet de connaître à chaque instant l'emplacement de chaque support 7 identifié. En effet, des moyens adaptés de détection peuvent être envisagés, comme des capteurs, permettant de lire les données inscrites sur chaque support 7, en entrée ou sortie, voire à l'intérieur des modules. Une telle lecture peut s'effectuer sans contact, de façon optique ou bien par ondes électromagnétiques, notamment par l'intermédiaire d'une puce RFID (pour « Radio Fréquence IDentification ») apposée sur chaque support 7 et d'au moins un lecteur adapté, placé à un ou plusieurs endroits du dispositif 1.

Une solution alternative peut consister uniquement en un compteur, sous forme d'un capteur mécanique ou numérique, incrémenté à chaque passage d'un support 7. En connaissant le nombre total de supports 7, il est alors possible de les identifier de façon unique en leur affectant un numéro.

La position de chaque support 7 au cours du traitement peut aussi être connue à partir de la position initiale lors du lancement et de la vitesse connue et régulée du convoyage. L'invention permet alors, au travers des moyens de gestion, de calculer l'emplacement exact à un instant précis de chaque support 7.

Dès lors, l'invention permet d'enregistrer les conditions de cuisson subies par chaque support 7 identifié à chaque étape dudit cycle, en fonction de ladite vitesse de convoyage. Pour ce faire, lesdits moyens de gestion comprennent des moyens d'enregistrement des conditions de cuisson subies par chaque support 7 au niveau de chaque section 6, en fonction de ladite vitesse d'avancement.

En d'autres termes, en calculant l'emplacement de chaque support 7 identifié à chaque instant, il est possible, au travers du contrôle des conditions appliquées à chaque étape, de déterminer le traitement exact qui est appliqué à chaque support 7 et, de ce fait, aux produits qu'il supporte.

En effet, l'invention consiste essentiellement à déterminer, au travers de moyens adaptés, les conditions de cuisson subies par les produits positionnés sur chaque support 7 identifié. Dès lors, il est possible de savoir, non plus théoriquement, ce que l'intégralité du lot de produits a subi comme traitement, avec les variations habituelles inhérentes dans ce type de procédé, mais d'extrapoler avec exactitude ce que chaque produit ou groupe de produits positionnés sur chaque support 7 a subi comme traitement à chaque étape du procédé.

Dès lors, il est possible d'intervenir sur chaque produit ou groupe de produits, indépendamment les uns des autres, en fonction des conditions réelles qu'ils ont chacun subies. En particulier, des alertes peuvent être déclenchées en cas de dysfonctionnement au cours de l'une ou l'autre étape du cycle de traitement, permettant de déterminer éventuellement quel produit n'a pas subi un traitement adapté et, grâce à l'identification du support 7 qui les transporte, de retirer ces produits en fin de cycle, à leur sortie du dispositif 1.

De façon connexe, étant donné que les modules 2, 3 sont constitués de colonnes 20, 30, l'invention prévoit de limiter l'interaction entre les différentes étapes et sections 6, améliorant ainsi la qualité et la précision du traitement effectué à chaque étape de cuisson. Pour ce faire, l'invention prévoit de canaliser l'écoulement de l'eau préalablement injectée par les moyens de chauffage et de refroidissement 5, afin qu'elle ne s'écoule pas vers les produits situés inférieurement. Pour ce faire, lesdits supports 7 comprennent des moyens de canalisation latérale de l'eau injectée par chaque buse 50, de sorte que l'eau ainsi canalisée s'écoule sur les côtés de chaque support 7. L'eau se retrouve alors déportée de part et d'autre et ne vient pas au contact des produits situés inférieurement. En d'autres termes, lesdits supports 7 munis des moyens de canalisation latérale de l'eau injectée par chaque buse 50 permet d'améliorer la qualité, d'ajuster et de contrôler avec précision les étapes de cuisson, en évitant que l'eau ne se retrouve projetée sur les produits des supports 7 situés inférieurement.

Cet aspect est notamment obtenu du fait que les supports 7 sont constitués d'une plaque pleine, empêchant d'être directement traversée par l'eau comme c'est le cas pour des plaques ajourées. Le fond plein permet de conserver l'eau et de n'autoriser l'écoulement qu'au niveau des bords latéraux, en particulier des moyens de canalisation latérale.

Cet écoulement est modélisé par des flèches sur la figure 2, représenté uniquement sur certains des supports 7 pour des raisons de clarté, mais l'écoulement s'effectue de façon similaire au niveau de chaque support 7 lorsqu'il reçoit de l'eau.

En outre, l'aspect plein permet une stagnation provisoire d'une quantité d'eau qui, dans le cas de produits ayant une surface de contact avec leur support 7, autorise une meilleure transmission thermique en partie inférieure de chaque produit placé sur son support 7.

De façon subsidiaire, l'invention vise l'utilisation spécifique et limitée au sein d'un dispositif 1 tel que précédemment décrit, d'au moins un support 7 sous forme de plaque pleine et pourvu de moyens de canalisation latérale d'eau s'écoulant sur ladite plaque. En d'autres termes, l'invention vise spécifiquement les moyens mis en œuvre pour chaque support 7 afin d'autoriser l'écoulement de chaque côté, afin de maintenir les conditions de traitement à chaque étape du procédé, permettant de s'assurer de la bonne cuisson des produits contenus sur chaque support 7, sans interférer avec les produits subissant une autre étape inférieurement.

De façon subsidiaire, l'invention vise l'utilisation pour la mise en œuvre du procédé tel que précédemment décrit, d'au moins un support 7 de produits identifié de façon unique. En d'autres termes, l'invention vise spécifiquement l'identification des supports 7 dans un cycle de traitement par cuisson, en vue de déterminer avec exactitude la position au cours du cycle de chaque produit ou groupe de produits d'un même lot et, de ce fait, à chaque étape, le traitement que chacun d'entre eux subit réellement.

## Revendications

1. Dispositif (1) de traitement par cuisson de produits, comprenant au moins un module de chauffage (2) et au moins un module de refroidissement (3), ainsi que des moyens de convoyage (8) desdits produits au travers desdits modules (2, 3), lesdits moyens de convoyage (8) comprenant des supports (7) sur lesquels sont positionnés lesdits produits, lesdits modules (2, 3) se présentant sous la forme d'au moins une colonne (20, 30) et comprenant réciproquement des moyens de chauffage et de refroidissement (5) situés au niveau des sections (6) de chaque colonne (20, 30), ledit dispositif (1) comprenant encore des moyens de gestion des conditions de cuisson de chacun desdits moyens de chauffage et de refroidissement (5), ainsi que de gestion de la vitesse d'avancement desdits moyens de convoyage (8),
**caractérisé par le fait que**
- chaque support (7) comprend un identifiant unique ;
- lesdits moyens de gestion comprennent des moyens d'enregistrement des conditions de cuisson subies par chaque support (7) au niveau de chaque section (6), en fonction de ladite vitesse d'avancement ;
- lesdits moyens de gestion comprenant des moyens de détermination des conditions de cuisson subies par lesdits produits positionnés sur chaque support (7) ;
- lesdits moyens de chauffage et de refroidissement (5) consistent en au moins des buses (50) d'injection d'eau et que lesdits supports (7) comprennent des moyens de canalisation latérale de l'eau injectée par chaque buse (50), de sorte que l'eau ainsi canalisée s'écoule sur les côtés de chaque support (7).

2. Dispositif (1) de traitement par cuisson selon la revendication précédente, **caractérisé en ce qu'**il comprend au moins un support (7) constitué d'une plaque pleine et pourvu de moyens de canalisation latérale d'eau, de sorte que l'eau canalisée s'écoule sur les côtés dudit support (7).

3. Procédé de traitement par cuisson de produits, mettant en œuvre le dispositif (1) selon l'une quelconque des revendications précédentes, dans lequel des produits subissent un cycle de cuisson sous forme d'au moins une étape de chauffage au sein d'au moins un module de chauffage (2) puis d'au moins une étape de refroidissement au sein d'au moins un module de refroidissement (3), lesdits produits étant disposés sur des supports (7) convoyés successivement au travers desdits modules de chauffage (2) et de refroidissement (3), ledit procédé consistant à gérer les conditions de cuisson de chaque étape de chauffage et de refroidissement en contrôlant au moins la température au niveau d'au moins une section (6) de chaque module (2, 3) et la vitesse de convoyage desdits supports (7),
**caractérisé en ce que** :
- on identifie de façon unique chaque support (7);
- on enregistre les conditions de cuisson subies par chaque support (7) identifié à chaque étape dudit cycle, en fonction de ladite vitesse de convoyage ;
- on détermine les conditions de cuisson subies par les produits positionnés sur chaque support (7) identifié.

4. Utilisation pour la mise en œuvre du procédé selon la revendication précédente, d'au moins un support (7) de produits identifié de façon unique.

## Patentansprüche

1. Vorrichtung (1) zur Verarbeitung von Produkten durch Garen, umfassend mindestens ein Heizmodul (2) und mindestens ein Kühlmodul (3) sowie Beförderungsmittel (8) der Produkte durch die Module (2, 3), wobei die Beförderungsmittel (8) Träger (7) umfassen, auf denen die Produkte positioniert sind, wobei die Module (2, 3) in Form von mindestens einer Säule (20, 30) vorliegen und reziprok Heiz- und Kühlmittel (5) umfassen, die sich im Bereich der Abschnitte (6) jeder Säule (20, 30) befinden, wobei die Vorrichtung (1) weiterhin Mittel zur Verwaltung der Garbedingungen jedes der Heiz- und Kühlmittel (5) sowie zur Verwaltung der Vortriebsgeschwindigkeit der Beförderungsmittel (8) umfasst,
**dadurch gekennzeichnet, dass**
- jeder Träger (7) eine einzige Kennung umfasst;
- die Verwaltungsmittel Speichermittel der Garbedingungen umfassen, denen jeder Träger (7) im Bereich jedes Abschnitts (6) ausgesetzt ist, in Abhängigkeit von der Vortriebsgeschwindigkeit;
- die Verwaltungsmittel Mittel zur Bestimmung der Garbedingungen umfassen, denen die auf jedem Träger (7) positionierten Produkte ausgesetzt sind;
- die Heiz- und Kühlmittel (5) aus mindestens Wasserspritzdüsen (50) bestehen und die Träger (7) seitliche Kanalisierungsmittel des von jeder Düse (50) gespritzten Wassers umfassen, so dass das derart kanalisierte Wasser über die Seiten jedes Trägers (7) fließt.

2. Vorrichtung (1) zur Verarbeitung durch Garen nach vorangehendem Anspruch, **dadurch gekennzeichnet, dass** sie mindestens einen Träger (7) umfasst, der aus einer vollen Platte besteht und mit seitlichen Wasserkanalisierungsmitteln versehen ist, so dass das kanalisierte Wasser über die Seiten des Trägers (7) fließt.

3. Verfahren zur Verarbeitung durch Garen von Produkten, das die Vorrichtung (1) nach einem der vorangehenden Ansprüche umsetzt, bei dem Produkte einem Garzyklus in Form von mindestens einem Heizschritt innerhalb von mindestens einem Heizmodul (2), dann von mindestens einem Kühlschritt innerhalb von mindestens einem Kühlmodul (3) unterzogen werden, wobei die Produkte auf Trägern (7) angeordnet sind, die nacheinander durch die Heiz- (2) und Kühlmodule (3) befördert werden, wobei das Verfahren darin besteht, die Garbedingungen jedes Heiz- und Kühlschritts durch Steuern von mindestens der Temperatur im Bereich von mindestens einem Abschnitt (6) jedes Moduls (2, 3) und der Beförderungsgeschwindigkeit der Träger (7) zu steuern,
**dadurch gekennzeichnet, dass**:
- jeder Träger (7) eindeutig identifiziert wird;
- die Garbedingungen, denen jeder identifizierte Träger (7) ausgesetzt ist, bei jedem Schritt des Zyklus identifiziert werden, in Abhängigkeit von der Beförderungsgeschwindigkeit;
- die Garbedingungen, denen die auf jedem identifizierten Träger (7) positionierten Produkte ausgesetzt sind, bestimmt werden.

4. Verwendung, für die Durchführung des Verfahrens nach vorangehendem Anspruch, von mindestens einem eindeutig identifizierten Produktträger (7).

## Claims

1. A device (1) for treating products by curing, comprising at least one heating module (2) and at least one cooling module (3), as well as conveying means (8) for conveying said products through said modules (2, 3), said conveying means (8) comprising supports (7) on which said products are positioned, said modules (2, 3) assuming the form of at least one column (20, 30) and reciprocally comprising heating and cooling means (5) located at sections (6) of each column (20, 30), said device (1) further comprising means for managing the curing conditions of each of said heating and cooling means (5), as well as for managing the speed of advance of said conveying means (8),
**characterized in that**
- each support (7) comprises a unique identifier;
- said management means comprise means for recording the curing conditions experienced by each support (7) at each section (6), based on said speed of advance;
- said management means comprise means for determining curing conditions experienced by said products positioned on each support (7);
- said heating and cooling means (5) consist of at least water injection nozzles (50) and said supports (7) comprise lateral channeling means of the water injected by each nozzle (50), such that the water thus channeled flows over the sides of each support (7).

2. The device (1) for treatment by curing according to the preceding claim, **characterized in that** it comprises at least one support (7) made up of a solid plate and provided with means for lateral water channeling, such that the channeled water flows over the sides of said support (7).

3. A treatment method by curing of products, implementing the device (1) according to any one of the preceding claims, wherein products undergo a curing cycle in the form of at least one heating step within at least one heating module (2), then at least one cooling step within at least one cooling module (3), said products being positioned on supports (7) successively conveyed through said heating (2) and cooling (3) modules, said method consisting of managing the curing conditions of each heating and cooling step by controlling at least the temperature at least at one section (6) of each module (2, 3) and the conveying speed of said supports (7),
**characterized in that**:
- each support (7) is identified uniquely;
- the curing conditions experienced by each support (7) identified in each step of said cycle are recorded, based on said conveying speed;
- the curing conditions experienced by the products positioned on each identified support (7) are determined.

4. A use for the implementation of the method according to the preceding claim, of at least one support (7) for products identified uniquely.
